# EUROPÄISCHE PATENTSCHRIFT

(11) **EP 2 916 764 B1**
(45) Veröffentlichungstag und Bekanntmachung des Hinweises auf die Patenterteilung: **19.12.2018**
(21) Anmeldenummer: 13792858.6
(22) Anmeldetag: 21.10.2013
(51) Int. Cl.: A61B 90/50

(54) **INSTRUMENTENHALTER ZUM BEFESTIGEN EINES MEDIZINISCHEN INSTRUMENTES AN EINEM GELENKARM**
INSTRUMENT HOLDER FOR MOUNTING A MEDICAL INSTRUMENT ON A JOINT ARM
SUPPORT D'INSTRUMENT SERVANT À FIXER UN INSTRUMENT MÉDICAL À UN BRAS ARTICULÉ

(30) Priorität: 09.11.2012 DE 102012110766
(43) Veröffentlichungstag der Anmeldung: 16.09.2015
(73) Patentinhaber: Maquet GmbH, 76437 Rastatt (DE)
(72) Erfinder: HAFNER, Dieter, Offenburg 77654 (DE); WYSLUCHA, Ulrich, 76356 Weingarten (DE)
(74) Vertreter: Zacco GmbH
(86) Internationale Anmeldenummer: PCT/EP2013/071948
(87) Internationale Veröffentlichungsnummer: WO 2014/072163

(56) Entgegenhaltungen:
- WO-A2-2008/128524
- US-A- 5 380 338
- US-A1- 2006 290 076
- US-A1- 2008 146 869
- US-A1- 2009 247 819
- US-A1- 2010 057 010
- US-A1- 2012 010 654
- US-A1- 2012 172 850

## Beschreibung

Die vorliegende Erfindung betrifft einen Instrumentenhalter zum Befestigen eines medizinischen Instrumentes, insbesondere eines Endoskops, an einem Gelenkarm, umfassend ein Gehäuse, eine an dem Gehäuse ausgebildete Halterung zum Halten des medizinischen Instrumentes und eine an dem Gehäuse ausgebildete Ankoppelvorrichtung zum Anbringen des Gehäuses an dem Gelenkarm.

Für chirurgische Anwendungen kommen heutzutage vermehrt Assistenzsysteme zum Einsatz, die einen Gelenkarm beinhalten, der beispielsweise an der Gleitschiene eines OP-Tisches anbringbar ist. An das freie Ende des frei beweglichen Gelenkarms wird ein medizinisches Instrument angekoppelt. Beispielsweise wird in der Laparoskopie ein mit einer Optik ausgestattetes starres Endoskop an dem Gelenkarm montiert und so ausgerichtet, dass das zu untersuchende oder chirurgisch zu versorgende Zielgebiet von dem Operateur eingesehen werden kann.

Um unterschiedliche Instrumente wahlweise mit dem Gelenkarm koppeln zu können, beinhalten solche Assistenzsysteme häufig einen Instrumentenhalter als mechanische Schnittstelle zwischen Instrument und Gelenkarm. Ein solcher Instrumentenhalter weist zum einen eine Ankoppelvorrichtung auf, die an dem freien Ende des Gelenkarms angebracht wird, sowie eine Halterung für das medizinische Instrument. Die Halterung ist beispielsweise als Schraubklemme ausgebildet und muss von dem Operateur oder einem Assistenten mit beiden Händen bedient werden, um das medizinische Instrument an dem Instrumentenhalter anzubringen. Die US 2012/172850 A1 offenbart einen Instrumentenhalter nach dem Oberbegriff des Anspruchs 1. Weiterer Stand der Technik ist aus US 2009/247819 A1 und US 2010/057010 A1 bekannt.

Aufgabe der Erfindung ist es, einen Instrumentenhalter anzugeben, der einfach aufgebaut und leicht zu bedienen ist.

Die Erfindung löst diese Aufgabe durch den Instrumentenhalter mit den Merkmalen des Anspruchs 1. Vorteilhafte Weiterbildungen sind in den Unteransprüchen angegeben.

Erfindungsgemäß weist die Halterung eine an einen Teil des medizinischen Instrumentes angepasste Aufnahmenut, in der dieser Instrumententeil aufnehmbar ist, und eine elastische Zuglasche auf, die an dem Gehäuse derart anbringbar ist, dass sie zum Halten des Instrumentes die Aufnahmenut zusammen mit dem in der Aufnahmenut angeordneten Instrumententeil umspannt.

Die Verwendung einer elastischen Zuglasche, die das Instrument in der Aufnahmenut festklemmt, hat gegenüber üblicherweise eingesetzten Schraubklemmen den Vorteil, dass man das an dem instrumentenhalter angebrachte Instrument innerhalb gewisser Grenzen nachjustieren kann, ohne das Instrument von dem Instrumentenhalter lösen zu müssen. Beispielsweise ist es möglich, das Instrument um seine Längsachse zu drehen, ohne die Zuglasche zu öffnen. Der erfindungsgemäße Instrumentenhalter ermöglicht so neben der erforderlichen Stabilität, mit der das Instrument zu halten ist, auch eine gewisse Flexibilität der Instrumentenbewegung und vereinfacht so die Handhabung erheblich.

Das Gehäuse des Instrumentenhalters, an dem zum einen die Halterung für das medizinische Instrument und zum anderen die Ankoppelvorrichtung zur Anbringung des Instrumentenhalters an dem Gelenkarm ausgebildet sind, kann beispielsweise in Form eines Kunststoff-Spritzgussteils kostengünstig hergestellt werden. Damit ist es möglich, den erfindungsgemäßen Instrumentenhalter als sterilen Einwegartikel zu konzipieren.

Um das medizinische Instrument an dem mit dem Gelenkarm gekoppelten Instrumentenhalter anzubringen, muss der Benutzer lediglich das Instrument in der Aufnahmenut anordnen und dann die Zuglasche unter Ausnutzung ihrer Elastizität über die Aufnahmenut ziehen und in geeigneter Weise an dem Gehäuse des Instrumentenhalters befestigen. Hierzu benötigt er nur eine Hand. Entsprechendes gilt für das Lösen des Instrumentes aus dem Instrumentenhalter. Die Erfindung ermöglicht so eine besonders einfache Einhandbedienung des Instrumentenhalters.

Vorzugsweise hat die Zuglasche einen ersten Laschenabschnitt, der auf einer Seite der Aufnahmenut angebracht ist, und einen zweiten Laschenabschnitt, der auf der anderen Seite der Aufnahmenut an dem Gehäuse unter elastischer Dehnung an der Zuglasche befestigbar ist, wodurch ein zwischen dem ersten und dem zweiten Laschenabschnitt liegender dritter Laschenabschnitt die Aufnahmenut in einem elastisch gedehnten Zustand umspannt. In einer besonders bevorzugten Ausgestaltung umspannt somit die Zuglasche die Aufnahmenut quer zur deren Längserstreckung, wobei der erste Laschenabschnitt auf einer Seite und der zweite Laschenabschnitt auf der anderen Seite der Aufnahmenut fixiert ist. Der Teil des Instrumentes, der in die Aufnahmenut eingepasst ist, wird auf diese Weise besonders zuverlässig arretiert.

Der zweite Laschenabschnitt ist vorzugsweise über eine Stift/Loch-Verbindung an dem Gehäuse befestigbar. Dadurch ist es möglich, die Zuglasche besonders einfach über die Aufnahmenut zu ziehen und an dem Gehäuse des Instrumentenhalters festzuspannen. Auch der erste Laschenabschnitt der Zuglasche lässt sich über eine entsprechende Stift/Loch-Verbindung an dem Gehäuse befestigen.

In einer vorteilhaften Ausführung hat der zweite Laschenabschnitt ein Befestigungsloch und das Gehäuse einen in das Befestigungsloch greifenden Befestigungsvorsprung, der an seinem freien Ende eine Arretiernase aufweist, die von der Aufnahmenut weg weist. Die Arretiernase weist demnach in eine Richtung, die der Zugspannung, die durch die elastische Verformung der Zuglasche erzeugt wird, entgegenwirkt. Dadurch ist sichergestellt, dass der zweite Laschenabschnitt der Zuglasche nicht von dem Befestigungsvorsprung abgleitet.

Eine entsprechende Ausgestaltung ist auch für die Anbringung des ersten Laschenabschnittes auf der anderen Seite der Aufnahmenut von Vorteil. In diesem Fall ist die Zuglasche als Ganzes von dem Gehäuse lösbar. Alternativ ist es ebenso möglich, den ersten Laschenabschnitt fest an dem Gehäuse anzubringen, so dass der zweite Laschenabschnitt das freie Ende einer an dem Gehäuse fest angebrachten Zuglasche darstellt.

Vorzugsweise bilden der erste, der zweite und der dritte Laschenabschnitt im entspannten Zustand der Zuglasche ein gerades, längliches Element. Dabei ist die Breite des länglichen Elementes vorzugsweise gleich der Länge der Aufnahmenut, über die sich die Zuglasche zum Halten des medizinischen Instrumentes erstreckt.

In einer besonders vorteilhaften Ausführung hat die Zuglasche einen an den dritten Laschenabschnitt anschließenden vierten Laschenabschnitt, der im entspannten Zustand der Zuglasche winklig, vorzugsweise rechtwinklig, von dem geraden Element absteht. Dieser vierte Laschenabschnitt bildet einen Griffteil, an dem der Benutzer die Zuglasche fasst, um letztere über die Aufnahmenut zu ziehen. Vorzugsweise ist die Oberfläche des vierten Laschenabschnittes geriffelt oder in anderer Weise oberflächenbehandelt, um ein Abgleiten der Finger des Benutzers von der Zuglasche zu verhindern.

Die Zuglasche ist vorzugsweise aus einem Elastomer, z.B. aus Gummi oder Silikon, gebildet. Es können beliebigen Materialien für die Zuglasche verwendet werden, sofern sichergestellt ist, das letztere die gewünschte Elastizität aufweist. Dabei liegt die Elastizität der Zuglasche beispielsweise in einem Shore-Härtebereich von 20 bis 100, vorzugsweise in einem Shore-Härtebereich von 30 bis 90 und besonders bevorzugt in einem Shore-Härtebereich von 45 bis 75. Die Materialdicke der Zuglasche liegt beispielsweise in einem Bereich von 1 bis 12 mm, vorzugsweise in einem Bereich von 2 bis 10 mm und besonders bevorzugt in einem Bereich von 3 bis 7 mm. Maßgeblich für die Bemessung der Elastizität der Zuglasche ist, dass die Zuglasche mit der Kraft einer Hand spannbar ist.

Die Aufnahmenut ist im Querschnitt vorzugsweise trapezförmig. Eine solche Trapezform ist besonders gut geeignet, das medizinische Instrument zu halten, da sie selbstzentrierend und toleranzausgleichend wirkt. Selbstverständlich ist die Aufnahmenut jedoch nicht auf einen trapezförmigen Querschnitt beschränkt. Es sind viele unterschiedliche Formen für die Aufnahmenut denkbar. Vorteilhaft ist es, wenn das in die Aufnahmenut geklemmte Instrument gegen zwei in Richtung des Nutbodens zulaufende Wände gedrückt wird. Aufgrund der auf diese Weise realisierten Keilwirkung kann mit einer kleinen Klemmkraft eine große Klemmwirkung erzielt werden. Dies ist beispielsweise auch mit einem dreieckigen Querschnitt oder einem halbellipsenförmigen Querschnitt möglich. Erfindungsgemäß ist die Aufnahmenut in einem elastischen Aufnahmestück ausgebildet, das in einer in dem Gehäuse ausgebildeten Passöffnung sitzt. Die Passöffnung bietet die Möglichkeit, unterschiedliche Aufnahmestücke für unterschiedliche medizinische Instrumente, die sich beispielsweise in ihrer Querschnittsform, insbesondere ihrem Durchmesser voneinander unterscheiden, als Instrumentenhalter anzubringen. Der Instrumentenhalter ist so für unterschiedliche Instrumente verwendbar.

Da das Aufnahmestück wie auch die Zuglasche elastisch ausgebildet ist, ermöglicht es eine gewisse Flexibilität bei der Ausrichtung des an dem Instrumentenhalter angebrachten medizinischen Instrumentes, ohne den Gelenkarm selbst bewegen zu müssen. Die Elastizität des Aufnahmestücks liegt dabei beispielsweise in einem Shore-Härtebereich von 20 bis 100, vorzugsweise in einem Shore-Härtebereich von 30 bis 90 und besonders bevorzugt in einem Shore-Härtebereich von 50 bis 75. Je weicher das Aufnahmestück ist, desto eher geht von dem Aufnahmestück ein gewisser "Klebeeffekt" aus, der die Fixierung des Instrumentes in der Aufnahmenut begünstigt.

Vorzugsweise greifen das Aufnahmestück und die Passöffnung nach Art einer Schwalbenschwanzverbindung ineinander. Eine Schwalbenschwanzverbindung ermöglicht eine besonders zuverlässige Anbringung des Aufnahmestücks in der Passöffnung.

In einer besonders bevorzugten Ausgestaltung ist das Gehäuse des Instrumentenhalters relativ zu der Ankoppelvorrichtung um eine Drehachse drehbar. Dies ermöglicht eine Drehung des an dem Instrumentenhalter angebrachten Instrumentes, ohne den Gelenkarm verstellen zu müssen.

In einer bevorzugten Ausführung umfasst die Ankoppelvorrichtung eine Rastplatte mit einer ersten Zahnung, die mit einer an dem Gehäuse ausgebildeten zweiten Zahnung in einem rastendem Eingriff steht, wobei die zweite Zahnung entgegen der von einem Vorspannelement ausgeübten Vorspannkraft längs der Drehachse aus der ersten Zahnung lösbar ist, um das Gehäuse relativ zu der Rastplatte um die Drehachse zu drehen. Die beiden Zahnungen können beispielsweise so ausgebildet sein, dass sich das Gehäuse in vorbestimmten Winkelintervallen relativ zu der Rastplatte drehen lässt.

Vorzugsweise ist der Instrumentenhalter derart ausgebildet, dass er sterilisierbar ist. In diesem Fall ist der Instrumentenhalter vorzugsweise ein Einwegartikel. Es ist jedoch ebenso möglich, den Instrumentenhalter mehrfach verwendbar, insbesondere mehrfach sterilisierbar auszubilden.

Nach einem weiteren Aspekt der Erfindung ist ein Assistenzsystem vorgesehen, das ein Gelenkarm, ein medizinisches Instrument und einen Instrumentenhalter vorstehend beschriebener Art zum Befestigen des medizinischen Instrumentes an dem Gelenkarm umfasst. Das medizinische Instrument ist beispielsweise ein Endoskop, das in der Laparoskopie eingesetzt wird, um dem Operateur die Möglichkeit zu geben, das zu behandelnde Zielgebiet einzusehen.

Die Erfindung wird im Folgenden anhand der Figuren näher erläutert. Darin zeigen:
Fig. 1 einen Gelenkarm, der Teil des erfindungsgemäßen Assistenzsystems ist;
Fig. 2 einen erfindungsgemäßen Instrumentenhalter, der an dem Gelenkarm nach Figur 1 anzubringen ist.
Fig. 3 eine Explosionsansicht des Instrumentenhalters;
Fig.4 eine perspektivische Ansicht des Gehäuses des Instrumentenhalters;
Fig.5 eine weitere perspektivische Ansicht des Gehäuses des Instrumentenhalters;
Fig.6 eine Ansicht des zusammengesetzten Gehäuses;
Fig.7 eine Ansicht des zusammengesetzten Gehäuses mit einem anderen Aufnahmestück;
Fig.8 eine Darstellung, die zeigt, wie das Gehäuse des Instrimentenhalters relativ zu der Rastplatte gedreht wird;
Fig.9 eine Darstellung, die zeigt, wie die Zuglasche um ein Endoskop gelegt wird; und
Fig. 10 eine Darstellung, die zeigt, wie das Endoskop durch die Zuglasche drehbar gehalten ist.

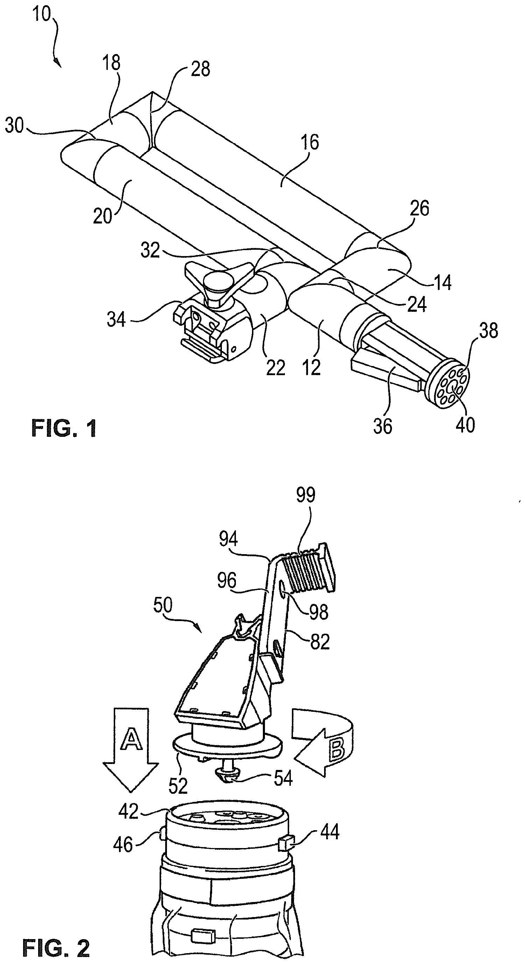

Figur 1 zeigt einen Gelenkarm 10, der Teil eines erfindungsgemäßen Assistenzsystems ist.

Der Gelenkarm 10 weist mehrere starre Halteglieder 12, 14, 16, 18, 20 und 22 auf, die über Gelenke 24, 26, 28, 30 und 32 miteinander gekoppelt sind. An einem Ende des Gelenkarms 10 ist eine Befestigungsvorrichtung 34 angeordnet, die dazu dient, den Gelenkarm 10 an einer nicht gezeigten Gleitschiene eines OP-Tisches anzubringen. An dem anderen Ende des Gelenkarms 10 befindet sich ein Handgriff 36, der von dem Benutzer manuell betätigt werden kann, um mit dem Gelenkarm 10 zu entriegeln.

Wird keine Betätigungskraft auf den Handgriff 36 ausgeübt, so sind die Halteglieder 12, 14, 16, 18, 20 und 22 des Gelenkarms 10 über die Gelenke 24, 26, 28, 30 und 32 starr miteinander gekoppelt. In diesem Zustand bildet der Gelenkarm eine starre Einheit.

Drückt der Benutzer den Handgriff 36, so werden die über die Gelenke 24, 26, 28, 30 und 32 miteinander gekoppelten Halteglieder 12, 14, 16, 18, 20 und 22 über eine Entriegelungsmechanik, die nicht Teil der vorliegenden Erfindung ist und deshalb an dieser Stelle nicht näher beschrieben wird, gegeneinander beweglich, so dass der Benutzer den Gelenkarm 10 nach Wunsch im Raum ausrichten kann. Lässt der Benutzer anschließend den Haltegriff 36 wieder los, so werden die Gelenke 24 bis 32 verriegelt, und der Gelenkarm 10 wird in seiner veränderten Ausrichtung fixiert.

Figur 2 zeigt einen Instrumentenhalter 50, der Teil des erfindungsgemäßen Assistenzsystems ist. Um den Instrumentenhalter 50 mit dem Gelenkarm 10 zu koppeln, ist eine Schnellkupplung 42 vorgesehen, die mit einem Ende auf einen Verriegelungszapfen 40 gesteckt wird, der an dem Handgriff 36 angeordnet ist (vgl. Figur 1). Die Schnellkupplung 42 hat zwei Entriegelungsknöpfe 44 und 46, die gedrückt werden, um die Schnellkupplung 42 von dem Gelenkarm 10 zu lösen.

Der Instrumentenhalter 50 ist auf die Schnellkupplung 42 adaptiert. Hierzu weist er eine Ankoppelvorrichtung 52 mit einem weiteren Verriegelungszapfen 54 auf, der in die Schnellkupplung 42 einsteckbar ist.

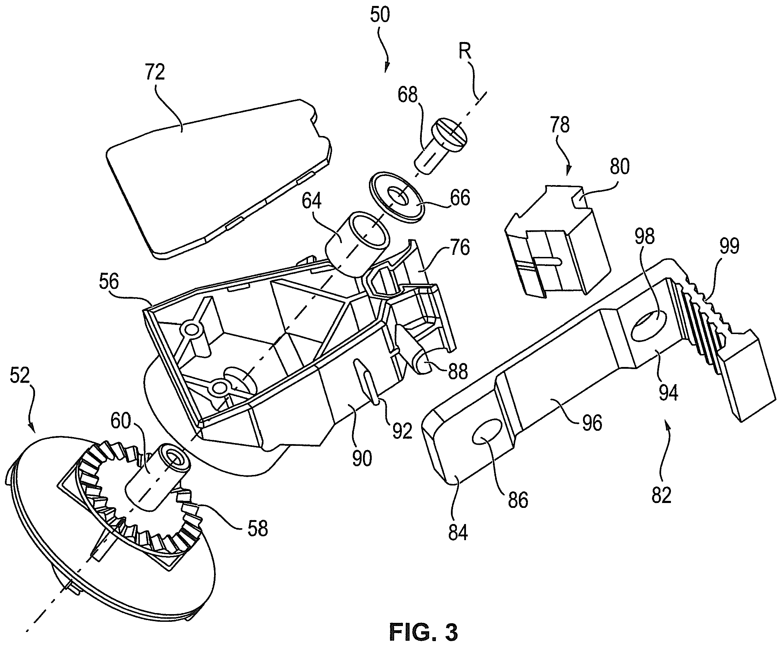

Der Instrumentenhalter 50 ist in Figur 3 in einer Explosionsansicht gezeigt.

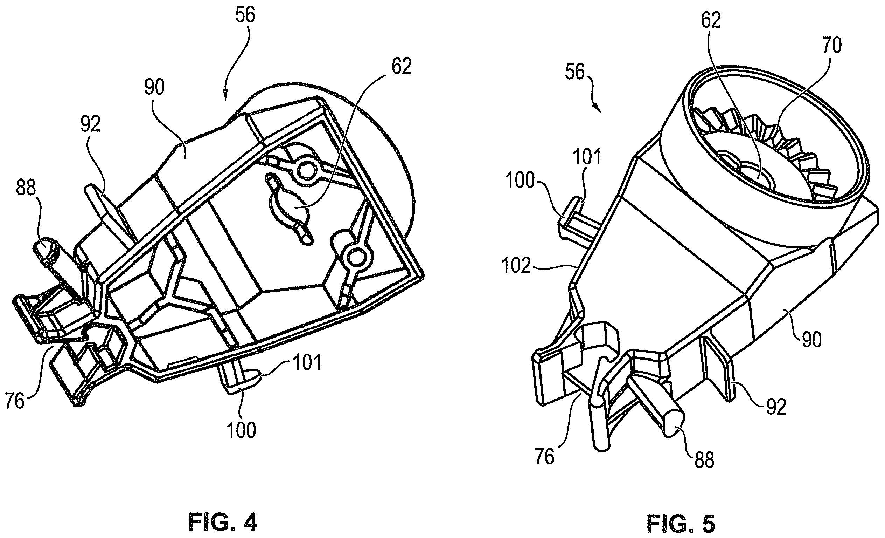

Der Instrumentenhalter 50 weist als Grundkomponente ein in Form eines einstückigen Kunststoff-Spritzgussteils gefertigtes Gehäuse 56 auf, das in den Figuren 4 und 5 in unterschiedlichen Perspektiven dargestellt ist.

Wie in der Explosionsdarstellung nach Figur 3 gezeigt, wird beim Zusammensetzen des Instrumentenhalters 50 die Ankoppelvorrichtung 52 an dem Gehäuse 56 angebracht. Die Ankoppelvorrichtung 52 ist aus einer kreisrunde Rastplatte gebildet, die auf ihrer dem Gehäuse 56 zugewandten Seite eine erste umlaufende Zahnung 58 aufweist. Innerhalb der Zahnung 58 befindet sich ein Gewindeschaft 60, der in eine in dem Gehäuse 56 ausgebildete Montageöffnung 62 (vgl. Fig. 4) eingesetzt wird. Ist der Gewindeschaft 60 in die Montageöffnung 62 eingesetzt, so werden nacheinander eine Druckschraubenfeder 64 und eine Unterlegscheibe 66 auf den Gewindeschaft 60 gesteckt. Anschließend wird eine Schraube 68 in den Gewindeschaft 60 geschraubt, wodurch die Rastplatte 52 entgegen der von der Druckschraubenfeder 64 ausgeübten Federkraft an dem Gehäuse 56 festgezogen wird.

Wie in Figur 5 gezeigt, weist das Gehäuse 56 eine zweite Zahnung 70 auf, die das Montageloch 62 umgibt. Die zweite Zahnung 70 des Gehäuses 56 befindet sich in Eingriff mit der auf der Rastplatte 52 ausgebildeten ersten Zahnung 58. Entgegen der von der Druckschraubenfeder 64 ausgeübten Federkraft lässt sich die Rastplatte 52 so weit von dem Gehäuse 56 abheben, dass die auf der Rastplatte 52 ausgebildete erste Zahnung 58 aus der in dem Gehäuse 56 ausgebildeten ersten Zahnung 70 gelöst wird. In diesem Zustand lässt sich das Gehäuse 56 um eine Drehachse R (vgl. Fig. 3), die mit der Mittelachse des Gewindeschafts 60 zusammenfällt, relativ zu der Rastplatte 52 drehen. Auf diese Weise lässt sich der Instrumentenhalter 50 relativ zu der Schnellkupplung 42 und damit zu dem Gelenkarm 10 drehen. Ein Deckel 72 verschließt das Gehäuse 56.

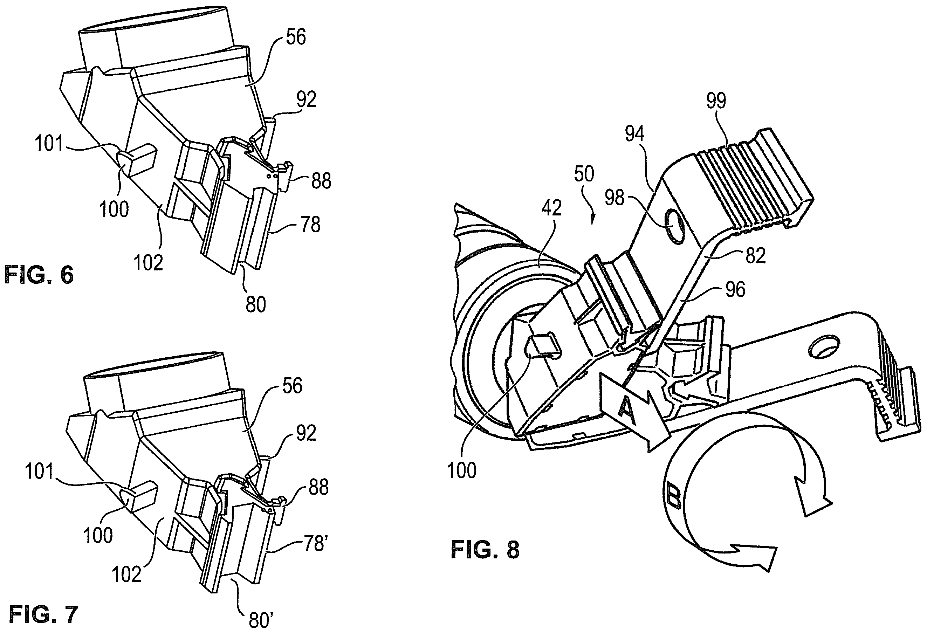

Das Gehäuse 56 weist an seinem der Rastplatte 52 abgewandten Ende eine Passöffnung 76 auf, in die ein elastisches Aufnahmestück 78 eingesetzt ist. Das Aufnahmestück 78 ist beispielsweise aus Gummi oder Silikon gefertigt und sitzt formschlüssig in der Passöffnung 76. Wie am besten in der Darstellung nach Figur 6 zu erkennen ist, greifen das Aufnahmestück 78 und die Passöffnung 76 nach Art einer Schwalbenschwanzverbindung formschlüssig ineinander.

Das Aufnahmestück 78 weist auf seiner von der Passöffnung 76 abgewandten Seite eine längliche, im Querschnitt trapezförmige Aufnahmenut 80 auf, die dazu dient, einen Teil des medizinischen Instrumentes, das mit dem Instrumentenhalter 50 gehalten werden soll, aufzunehmen. Dementsprechend sind Form und Größe der Aufnahmenut 80 an diesen Instrumententeil angepasst.

Wie den Darstellungen nach den Figuren 6 und 7 zu entnehmen ist, können unterschiedliche elastische Aufnahmestücke 78, 78' bereitgehalten werden, deren jeweilige Aufnahmenut 80, 80' an das gerade verwendete medizinische Instrument angepasst ist. Hierzu sind diejenigen Teile der Aufnahmestücke 78, 78', die an die Passöffnung 76 angepasst sind und mit dieser die Schwalbenschwanzverbindung bilden, identisch ausgebildet. Nur die Aufnahmenuten 80, 80' sind unterschiedlich. Der Instrumentenhalter 50 umfasst ferner eine elastische Zuglasche 82, die dazu dient, den in die Aufnahmenut 80 des Befestigungsstücks 78 eingelegten Instrumententeil in der Aufnahmenut 80 festzuklemmen. Die Zuglasche 82, die z.B. aus Gummi oder Silikon gefertigt ist, hat einen ersten Laschenabschnitt 84, in dem ein Befestigungsloch 86 ausgebildet ist. Der erste Laschenabschnitt 84 wird mit seinem Befestigungsloch 86 auf einen Befestigungsvorsprung 88 gesteckt, so dass der erste Laschenabschnitt 84 an einer Außenwand 90 des Gehäuses 56 festgesetzt ist. Hierzu ist der Befestigungsvorsprung 88 so breit, dass das Befestigungsloch 86, wenn es auf den Befestigungsvorsprung 88 gesteckt ist, elastisch verformt wird, wodurch der erste Laschenabschnitt 84 auf dem Befestigungsvorsprung 88 fixiert wird. In diesem fixierten Zustand liegt die Endfläche des ersten Laschenabschnittes 84 an einem Anlagevorsprung 92 an, der senkrecht von der Gehäusewand 90 des Gehäuses 56 absteht.

Die Zuglasche 82 hat einen zweiten Laschenabschnitt 94 und einen dritten Laschenabschnitt 96, der zwischen dem ersten Laschenabschnitt 84 und dem zweiten Laschenabschnitt 94 angeordnet ist. In dem zweiten Laschenabschnitt 94 ist ein zweites Befestigungsloch 98 ausgebildet, das auf einen zweiten Befestigungsvorsprung 100 (vgl. Figuren 4 und 5) gesteckt wird. Der zweite Befestigungsvorsprung 100 hat eine Rastnase 101, die von der Aufnahmenut 80 weg weist. Er befindet sich an einer Gehäusewand 102, die von der Gehäusewand 90, an welcher der erste Befestigungsvorsprung 88 ausgebildet ist, abgewandt ist. Die beiden Befestigungsvorsprünge 88 und 100 befinden sich somit auf verschieden Seiten der Aufnahmenut 80.

Der zweite Laschenabschnitt 94 wird mit seinem Befestigungsloch 98 auf den Befestigungsvorsprung 100 gesteckt, um die Zuglasche 82 so an dem Gehäuse 56 anzubringen, dass diese mit ihrem dritten Laschenabschnitt 96 die in dem Befestigungsstück 78 ausgebildete Aufnahmenut 80 mitsamt dem darin gehaltenen Instrumententeil umspannt und dabei in ihrer Längsrichtung elastisch auseinander gezogen wird. Durch die elastische Verformung der Zuglasche 82 drückt der die Aufnahmenut 80 umspannende dritte Laschenabschnitt 96 den Instrumententeil in die Aufnahmenut 80.

Die Zuglasche 82 hat ferner einen vierten Laschenabschnitt 99, der in einem rechten Winkel an den zweiten Laschenabschnitt 94 anschließt. Der vierte Laschenabschnitt 99 hat eine geriffelte Oberfläche und bildet einen Griffteil, an dem der Benutzer die Zuglasche 82 fassen kann.

Wie vorstehend erwähnt, befinden sich die beiden Befestigungsvorsprünge 88 und 100 auf verschiedenen Seiten der Passöffnung 76 und damit der in dem Befestigungsstück 78 ausgebildeten Aufnahmenut 80. Die Strecke, über welche sich die Zuglasche 82 ausgehend von dem ersten Befestigungsvorsprung 88 über die Aufnahmenut 80 hinweg bis zu dem zweiten Befestigungsvorsprung 100 erstreckt, ist kürzer als der Abstand, den die beiden Befestigungslöcher 86 und 98 im entspannten Zustand der Zuglasche 82 voneinander haben. Dadurch ist sichergestellt, dass die Zuglasche 82 im gespannten Zustand, in dem der erste Befestigungsvorsprung 88 in das erste Befestigungsloch 86 und der zweite Befestigungsvorsprung 100 in das zweite Befestigungsloch 98 greift, hinreichend gedehnt ist, um infolge ihrer Elastizität den Instrumententeil mit hinreichend großer Haltekraft in die Aufnahmenut 80 zu drücken.

Im Folgenden wird die Funktionsweise des Instrumentenhalters 5 unter Bezugnahme auf die Figuren 2, 8, 9 und 10 erläutert.

Zur Anbringung des Instrumentenhalters 50 an der mit dem Gelenkarm 10 verbundenen Schnellkupplung 40 wird der Verrieglungszapfen 54, der an der Rastplatte 52 ausgebildet ist, im Pfeilrichtung A (vgl. Figur 2) in die Schnellkupplung 42 gesteckt. Wird der Instrumentenhalter 50 anschließend in die Pfeilrichtung B gedreht, so rastet er hörbar ein und ist mit der Schnellkupplung 42 verriegelt.

Figur 8 zeigt, wie der verriegelte Instrumentenhalter 50 relativ zu der Schnellkupplung 42 verdreht werden kann, um den Instrumentenhalter 50 beispielsweise an einem schon ausgerichteten Endoskop anzubringen. Hierzu wird das Gehäuse 56 in Pfeilrichtung A entgegen der von der Druckschraubenfeder 64 ausgeübten Federkraft etwas von der Rastplatte 52 abgehoben, so dass sich die beiden Zahnungen 58 und 70 voneinander lösen. In diesem gelösten Zustand kann das Gehäuse 56 relativ zu der Rastplatte 52 gedreht werden, wie in Figur 8 durch den Pfeil B angedeutet ist. Zur Veranschaulichung dieses Vorgangs ist der Instrumentenhalter 50 in Figur 8 in zwei Drehstellungen gezeigt.

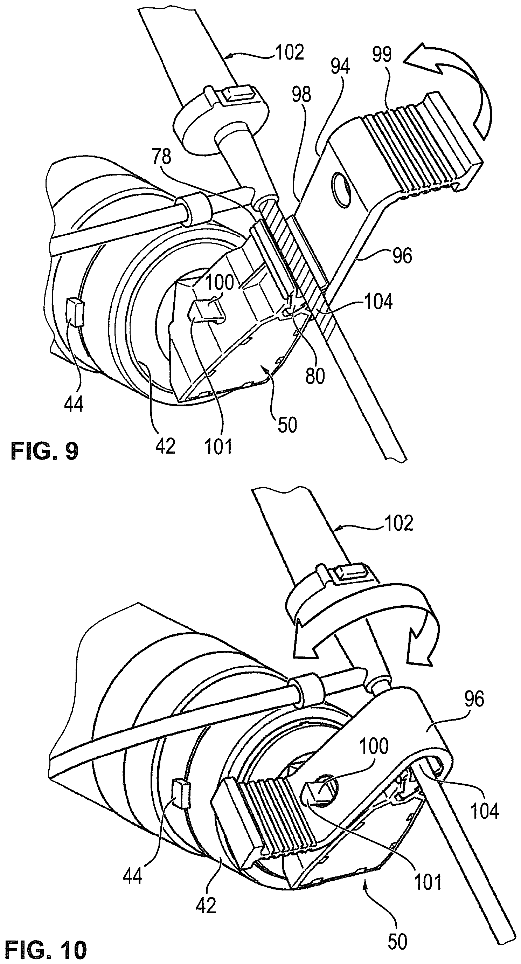

In Figur 9 ist dargestellt, wie ein Endoskop 102 an dem Instrumentenhalter 50 angebracht wird. Hierzu wird ein Rohrabschnitt 104 des Endoskops 102 in die Aufnahmenut 80 des Befestigungsstücks 78 eingelegt. Anschließend fasst die Bedienperson den geriffelten Griffteil 99 der Zuglasche 82 und klappt die Zuglasche 82 um, wie in Figur 9 durch den Pfeil angedeutet ist.

Schließlich klemmt der Benutzer den Rohrabschnitt 104 des Endoskops 102 in der Aufnahmenut 80 fest, indem er den Befestigungsvorsprung 100 in dem Befestigungsloch 98 der Zuglasche 82 einhakt, wie in Figur 10 gezeigt ist. Der Darstellung nach Figur 10 ist auch zu entnehmen, dass sich das Endoskop 102 um seine Längsachse drehen lässt, obgleich es durch die Zuglasche 82 in der Aufnahmenut 80 gehalten ist. Die Zuglasche 82 ermöglicht demnach eine gebremste Drehbewegung des in der Aufnahmenut 80 angeordneten Endoskops 102, ohne die Zuglasche 82 lösen zu müssen. Im statischen Betrieb, in dem keine äußere Kraft auf das Endoskop wirkt, ist letzteres somit durch die Zuglasche 82 sicher in der Aufnahmenut 80 gehalten. Im dynamischen Betrieb, in dem der Benutzer eine Drehkraft auf das Endoskop 102 ausübt, erlaubt demgegenüber die Zuglasche 82 eine Drehbewegung des Endoskops 102 um seine Längsachse. Letzteres ist insbesondere bei der Verwendung von Winkeloptiken von Vorteil. Ein aktives Lösen des Endoskops, wie dies beispielsweise bei herkömmlichen Schraubklemmen vorgesehen ist, ist demnach nicht erforderlich.

## Patentansprüche

1. Instrumentenhalter (50) zum Befestigen eines medizinischen Instrumentes (102) an einem Gelenkarm (10),
**dadurch gekennzeichnet, dass**
der Instrumentenhalter (50) umfasst:
ein Gehäuse (56),
eine an dem Gehäuse (56) ausgebildete Halterung zum Halten des medizinischen Instrumentes (102), und
eine an dem Gehäuse (56) ausgebildete Ankoppelvorrichtung (52) zum Anbringen des Gehäuses (56) an dem Gelenkarm (10),
wobei die Halterung eine an einen Teil (104) des medizinischen Instrumentes (102) angepasste Aufnahmenut (80) umfasst, in der dieser Instrumententeil (104) aufnehmbar ist, und eine elastische Zuglasche (82) aufweist, die an dem Gehäuse (56) derart anbringbar ist, dass sie zum Halten des Instrumentes (102) die Aufnahmenut (80) mitsamt dem in der Aufnahmenut (80) angeordneten Instrumententeil (104) umspannt, und wobei die Aufnahmenut (80) in einem elastischen Aufnahmestück (78) ausgebildet ist, das herausnehmbar in einer in dem Gehäuse (56) ausgebildeten Passöffnung (76) sitzt.

2. Instrumentenhalter (50) nach Anspruch 1, **dadurch gekennzeichnet, dass** die Zuglasche (82) einen ersten Laschenabschnitt (84), der auf einer Seite der Aufnahmenut (80) angebracht ist, und einen zweiten Laschenabschnitt (94) hat, der auf der anderen Seite der Aufnahmenut (80) an dem Gehäuse (56) unter elastischer Dehnung der Zuglasche (82) befestigbar ist, wodurch ein zwischen dem ersten und dem zweiten Laschenabschnitt (84, 94) liegender dritter Laschenabschnitt (96) die Aufnahmenut (80) in einem elastisch gedehnten Zustand umspannt.

3. Instrumentenhalter (50) nach Anspruch 2, **dadurch gekennzeichnet, dass** der zweite Laschenabschnitt (94) über eine Stift/Loch-Verbindung (98, 100) an dem Gehäuse (56) befestigbar ist.

4. Instrumentenhalter (50) nach Anspruch 3, **dadurch gekennzeichnet, dass** der zweite Laschenabschnitt (94) ein Befestigungsloch (98) und das Gehäuse (56) einen in das Befestigungsloch (98) greifenden Befestigungsvorsprung (100) hat, der an seinem freien Ende eine Arretiernase (101) aufweist, die von der Aufnahmenut (80) weg weist.

5. Instrumentenhalter (50) nach einem der Ansprüche 2 bis 4, **dadurch gekennzeichnet, dass** der erste, der zweite und der dritte Laschenabschnitt (84, 94, 96) im entspannten Zustand der Zuglasche (82) ein gerades, längliches Element bilden.

6. Instrumentenhalter (50) nach Anspruch 5, **dadurch gekennzeichnet, dass** die Zuglasche (82) einen an den zweiten Laschenabschnitt (94) anschließenden vierten Laschenabschnitt (99) hat, der im entspannten Zustand der Zuglasche (82) winklig von dem geraden Element absteht.

7. Instrumentenhalter (50) nach einem der vorhergehenden Ansprüche, **dadurch gekennzeichnet, dass** die Zuglasche (82) aus einem Elastomer gebildet ist.

8. Instrumentenhalter (50) nach einem der vorhergehenden Ansprüche, **dadurch gekennzeichnet, dass** die Zuglasche (82) den Instrumententeil (104) derart in der Aufnahmenut (80) hält, dass sich der Instrumententeil (104) entgegen der von der Zuglasche (82) ausgeübten Haltekraft um seine Längsachse drehen lässt.

9. Instrumentenhalter (50) nach einem der vorhergehenden Ansprüche, **dadurch gekennzeichnet, dass** das Aufnahmestück (78) und die Passöffnung (76) nach Art einer Schwalbenschwanzverbindung ineinander greifen.

10. Instrumentenhalter (50) nach einem der vorhergehenden Ansprüche, **dadurch gekennzeichnet, dass** das Aufnahmestück (78) aus einem Elastomer gebildet ist.

11. Instrumentenhalter (50) nach einem der vorhergehenden Ansprüche, **dadurch gekennzeichnet, dass** das Gehäuse (56) relativ zu der Ankoppelvorrichtung (52) um eine Drehachse (R) drehbar ist.

12. Instrumentenhalter (50) nach Anspruch 11, **dadurch gekennzeichnet, dass** die Ankoppelvorrichtung eine Rastplatte (52) mit einer ersten Zahnung (58) umfasst, die mit einer an dem Gehäuse (56) ausgebildeten zweiten Zahnung (70) in rastendem Eingriff steht, wobei die zweite Zahnung (70) entgegen der von einem Vorspannelement (64) ausgeübten Vorspannkraft längs der Drehachse (R) aus der ersten Zahnung (58) lösbar ist, um das Gehäuse (56) relativ zu der Rastplatte um die Drehachse (R) zu drehen.

13. Instrumentenhalter (50) nach Anspruch 12, **dadurch gekennzeichnet, dass** die Rastplatte (52) mittels einer Schraubverbindung, die aus einem Gewindeschaft (60) und einem in den Gewindeschaft (60) eingesetzten Schraubelement (68) gebildet ist, an dem Gehäuse (56) befestigt und mittels einer auf Druck belasteten Feder (64), in die der Gewindeschaft eingesetzt (60) ist, gegen das Gehäuse (56) gedrückt ist.

14. Gelenkarmsystem, umfassend einen Gelenkarm (10), ein medizinisches Instrument, vorzugsweise ein Endoskop (102), und einen Instrumentenhalter (50) nach einem der vorhergehenden Ansprüche zum Befestigen des medizinischen Instrumentes an dem Gelenkarm (10).

## Claims

1. An instrument holder (50) for mounting a medical instrument (102) on a joint arm (10)
**characterized in that**
the instrument holder (50) comprises:
a housing (56),
a holder formed on the housing (56) in order to hold the medical instrument (102), and
a coupling device (52) formed on the housing (56) in order to attach the housing (56) to the joint arm (10),
wherein the holder comprises a receiving groove (80) adapted to a part (104) of the medical instrument (102), in which receiving groove said instrument part (104) is receivable, and an elastic pull strap (82) that is attachable to the housing (56) in such a manner that it surrounds the receiving groove (80) together with the instrument part (104) arranged in the receiving groove (80) in order to hold the instrument (102), and wherein the receiving groove (80) is formed in an elastic receiving piece (78), which rests removably in a fitting opening (76) formed in the housing (56) .

2. The instrument holder (50) according to claim 1, **characterized in that** the elastic pull strap (82) has a first strap portion (84), which is attached on one side of the receiving groove (80), and a second strap portion (94), which is mountable on the other side of the receiving groove (80) on the housing (56) under elastic expansion of the pull strap (82), whereby a third strap portion (96) positioned between the first and the second strap portion (84, 94) surrounds the receiving groove (80)in an elastically expanded state.

3. The instrument holder (50) according to claim 2, **characterized in that** the second strap portion (94) is mountable via a pin/hole connection (98, 100) on the housing (56).

4. The instrument holder (50) according to claim 3, **characterized in that** the second strap portion (94) has a mounting hole (98) and the housing (56) has a mounting projection (100) engaging in the mounting hole (98), which mounting projection includes on its free end a locking nose (101) which points away from the receiving groove (80).

5. The instrument holder (50) according to one of claims 2 to 4, **characterized in that** the first, second and third strap portions (84, 94, 96), in the untensioned state of the pull strap (82), form a straight, elongated element.

6. The instrument holder (50) according to claim 5, **characterized in that** the pull strap (82) has a fourth strap portion (99) adjacent to the second strap portion (94), which extends at an angle from the straight element in the untensioned state of the pull strap (82) .

7. The instrument holder (50) according to one of the preceding claims, **characterized in that** the pull strap (82) is made of an elastomer.

8. The instrument holder (50) according to one of the preceding claims, **characterized in that** the pull strap (82) holds the instrument part (104) in the receiving groove (80) in such a manner that the instrument part (104) is rotatable around its longitudinal axis against the holding force exerted by the pull strap (82).

9. The instrument holder (50) according to one of the preceding claims, **characterized in that** the receiving piece (78) and the fitting opening (76) engage in the manner of a dovetail joint connection.

10. The instrument holder (50) according to one of the preceding claims, **characterized in that** the receiving piece (78) is made of an elastomer.

11. The instrument holder (50) according to one of the preceding claims, **characterized in that** the housing (56) is rotatable relative to the coupling device (52) around a rotational axis (R).

12. The instrument holder (50) according to claim 11, **characterized in that** the coupling device comprises a locking plate (52) having a first toothing (58) engaged in an interlocking manner with a second toothing (70) formed on the housing (56), wherein the second toothing (70) is detachable from the first toothing (58) against the biasing force exerted by a biasing element (64) along the rotational axis (R), in order to rotate the housing (56) relative to the locking plate around the rotational axis (R).

13. The instrument holder (50) according to claim 12, **characterized in that** the locking plate (52) is mounted on the housing (56) by means of a screw connection, which is formed from a threaded shank (60) and a screw element (68) inserted in the threaded shank (60), and is pressed against the housing (56) by means of a compression spring (64) in which the threaded shank (60) is inserted.

14. A joint arm system, comprising a joint arm (10), a medical instrument, preferably an endoscope (102), and an instrument holder (50) according to one of the preceding claims for mounting the medical instrument on the joint arm (10).

## Revendications

1. Support d'instrument (50) servant à fixer un instrument médical (102) à un bras articulé (10),
**caractérisé en ce que**
le support d'instrument (50) comprend :
un boîtier (56),
un dispositif de retenue réalisé au niveau du boîtier (56) pour retenir l'instrument médical (102), et
un dispositif d'accouplement (52) réalisé au niveau du boîtier (56) pour le montage du boîtier (56) sur le bras articulé (10),
dans lequel le dispositif de retenue comprend une rainure de réception (80) adaptée à une partie (104) de l'instrument médical (102), dans laquelle cette partie d'instrument (104) peut être reçue, et présente une languette de traction élastique (82), qui peut être montée sur le boîtier (56) de telle sorte qu'elle enserre la rainure de réception (80) avec la partie d'instrument (104) agencée dans la rainure de réception (80) pour retenir l'instrument (102), et dans lequel la rainure de réception (80) est réalisée dans une pièce de réception élastique (78), qui est logée de manière amovible dans une ouverture ajustée (76) réalisée dans le boîtier (56).

2. Support d'instrument (50) selon la revendication 1, **caractérisé en ce que** la languette de traction (82) a une première section de languette (84), qui est montée d'un côté de la rainure de réception (80), et une deuxième section de languette (94), qui peut être fixée de l'autre côté de la rainure de réception (80) sur le boîtier (56) sous allongement élastique de la languette de traction (82), moyennant quoi une troisième section de languette (96) située entre la première et la deuxième section de languette (84, 94) enserre la rainure de réception (80) dans un état d'allongement élastique.

3. Support d'instrument (50) selon la revendication 2, **caractérisé en ce que** la deuxième section de languette (94) peut être fixée au boîtier (56) par le biais d'une liaison tige/trou (98, 100).

4. Support d'instrument (50) selon la revendication 3, **caractérisé en ce que** la deuxième section de languette (94) a un trou de fixation (98) et le boitier (56) a une saillie de fixation (100) venant en prise avec le trou de fixation (98), qui présente à son extrémité libre un nez d'arrêt (101), qui s'éloigne de la rainure de réception (80).

5. Support d'instrument (50) selon l'une quelconque des revendications 2 à 4, **caractérisé en ce que** la première, la deuxième et la troisième section de languette (84, 94, 96) forment à l'état détendu de la languette de traction (82), un élément droit allongé.

6. Support d'instrument (50) selon la revendication 5, **caractérisé en ce que** la languette de traction (82) a une quatrième section de languette (99) se raccordant à la deuxième section de languette (94), qui à l'état détendu de la languette de traction (82) fait saillie de façon angulaire de l'élément droit.

7. Support d'instrument (50) selon l'une quelconque des revendications précédentes, **caractérisé en ce que** la languette de traction (82) est formée d'un élastomère.

8. Support d'instrument (50) selon l'une quelconque des revendications précédentes, **caractérisé en ce que** la languette de traction (82) retient la partie d'instrument (104) dans la rainure de réception (80) de telle sorte que la partie d'instrument (104) se laisse tourner dans le sens opposé à la force de retenue exercée par la languette de traction (82) autour de son axe longitudinal.

9. Support d'instrument (50) selon l'une quelconque des revendications précédentes, **caractérisé en ce que** la pièce de réception (78) et l'ouverture ajustée (76) s'imbriquent l'une dans l'autre à la manière d'une liaison en queue d'aronde.

10. Support d'instrument (50) selon l'une quelconque des revendications précédentes, **caractérisé en ce que** la pièce de réception (78) est formée d'un élastomère.

11. Support d'instrument (50) selon l'une quelconque des revendications précédentes, **caractérisé en ce que** le boîtier (56) est rotatif par rapport au dispositif d'accouplement (52) autour d'un axe de rotation (R).

12. Support d'instrument (50) selon la revendication 11, **caractérisé en ce que** le dispositif d'accouplement comprend une plaque d'arrêt (52) avec une première denture (58), qui est en prise d'arrêt avec une deuxième denture (70) réalisée au niveau du boîtier (56), dans lequel la deuxième denture (70) est détachable de la première denture (58) dans le sens opposé à la force de précontrainte exercée par un élément de précontrainte (64) le long de l'axe de rotation (R) pour tourner le boîtier (56) par rapport à la plaque d'arrêt autour de l'axe de rotation (R).

13. Support d'instrument (50) selon la revendication 12, **caractérisé en ce que** la plaque d'arrêt (52) est fixée au boîtier (56) au moyen d'un raccord vissé, qui est formé d'une tige filetée (60) et d'un élément à vis (68) inséré dans la tige filetée (60), et est pressée contre le boîtier (56) au moyen d'un ressort (64) sollicité en pression, dans lequel la tige filetée (60) est insérée.

14. Système de bras articulé, comprenant un bras articulé (10), un instrument médical, de préférence un endoscope (102), et un support d'instrument (50) selon l'une quelconque des revendications précédentes pour la fixation de l'instrument médical au bras articulé (10).
